# EUROPÄISCHE PATENTSCHRIFT

(11) **EP 1 360 948 B1**
(45) Veröffentlichungstag und Bekanntmachung des Hinweises auf die Patenterteilung: **14.01.2004**
(21) Anmeldenummer: 02010047.5
(22) Anmeldetag: 06.05.2002
(51) Int. Cl.: A61F 2/30, A61F 2/46

(54) **Vorrichtung zum Zuschneiden von Knorpeln**
Device for shaping cartilage grafts
Dispositif pour former un greffon de cartilage

(43) Veröffentlichungstag der Anmeldung: 12.11.2003
(73) Patentinhaber: Karl Storz GmbH & Co. KG, 78532 Tuttlingen (DE)
(72) Erfinder: Efinger, Andreas, 78604 Rietheim (DE)
(74) Vertreter: Weller, Wolfgang, Dr.

(56) Entgegenhaltungen:
- EP-A- 1 059 153
- WO-A-95/30742

## Beschreibung

Die Erfindung betrifft eine Vorrichtung zum Zuschneiden von Knorpeln, mit einem ersten Körper und einem zweiten Körper, zwischen die der zu schneidende Knorpel legbar ist, und mit einer Aufnahme, in der der Knorpel aufnehmbar ist.

Eine derartige Vorrichtung ist aus der DE 40 34 615 A1 bekannt.

Solche medizinischen Schneidevorrichtungen dienen dazu, einen Knorpel auf eine bestimmte Größe zuzuschneiden.

Bei manchen chirurgischen Eingriffen werden dünne Knorpelscheiben benötigt, beispielsweise zur plastischen Versorgung eines Trommelfelldefektes. Die Knorpelstücke, die von anderen Organen, beispielsweise dem Nasenseptum, entnommen werden, sind für diesen Zweck zu dick und müssen daher auf eine gewünschte Größe und Form zugeschnitten werden.

Die freihändige Präparation mit Pinzette und Schere oder Skalpell ist äußerst schwierig, so daß man derartige medizinische Schneidevorrichtungen zu Hilfe nimmt.

Bei der eingangs genannten Vorrichtung ist in dem ersten Körper eine zu einer Stirnseite des Körpers hin offene Aufnahme ausgespart. Der zweite Körper, der auf den ersten auflegbar ist, schließt diese Aufnahme als Deckel ab. In den so geschaffenen zur Stirnseite hin offenen Raum wird der Knorpel eingebracht.

In einem vorgegebenen Abstand parallel zur Grundfläche der Aufnahme ist ein Führungsschlitz vorgesehen, der als Führung für eine Schneideklinge, beispielsweise in Form einer Rasierklinge, dient. Dadurch kann der in der Aufnahme zwischen dem ersten und zweiten Körper gehaltene Knorpel von der Außenseite her durch Einbringen der Schneideklinge in zwei Teile getrennt, also zugeschnitten, werden. Durch Einlegen maßgenauer Distanzblättchen in die Vertiefung können entsprechend dünnere Knorpelscheiben erhalten werden.

Diese Vorrichtung weist, insbesondere was die Variabilität betrifft, Nachteile dahingehend auf, daß zur Änderung der gewünschten Knorpeldicke feinste Distanzblättchen in die ohnehin schon kleine Aufnahme eingebracht werden müssen. Es ist zu bedenken, daß beispielsweise bei der Beseitigung von Trommelfelldefekten diese Implantate eine Größe im Bereich von Millimetern haben.

Wenn nun unterschiedliche Größen und unterschiedliche Formen an Knorpeln gewünscht werden, je nach der jeweiligen Anatomie des Patienten, müssen dementsprechend unterschiedlich ausgebildete Vorrichtungen mit den entsprechenden zahlreichen Distanzblättchen bereitgehalten werden.

Bei der Reparatur von Trommelfelldefekten ist es wünschenswert, um den Knorpel herum noch eine gewisse Menge der Knorpelhaut zu erhalten. Dies dient dazu, das Transplantat mittels der Knorpelhaut mit dem eigentlichen Trommelfell zu verbinden, von dem der zugeschnittene Knorpel vorsteht, der als Auflage für den Hammer, den sog. Gehörknochen, dient, über den die Schwingungen des Trommelfells nach der Reparatur aufgenommen werden.

Bei der eingangs genannten Vorrichtung ist es nicht möglich und auch nicht vorgesehen, den Knorpel umgebende Knorpelhaut am Knorpel zu belassen.

Aus der EP-A-1 059 153 ist eine Fertigungsform zur industriellen und intraoperativen Herstellung von implantierfähigen Fertigstücken aus Knochenersatzmaterialien bekannt. Diese Form weist eine Trägerplatte, einen Formrahmen mit einer zentralen Aussparung für die Aufnahme des zu implantierenden Knochenersatzmaterials und eine Deckelplatte auf. Diese sind während des Abbindevorgangs des ursprünglich pastösen Knochenersatzmaterials passgenau übereinander angeordnet. Die Form des herstellbaren Knochenersatzimplantates wird durch einen ebenen oder gekrümmten Formrahmen mit einer im Wesentlichen beispielsweise kreisförmigen rechteckigen oder dreieckigen Aussparung bestimmt.

Es ist Aufgabe der vorliegenden Erfindung, eine Vorrichtung zum Zuschneiden von Knorpeln der eingangs genannten Art dahingehend zu verbessern, dass mit robusten und gut handhabbaren Bauteilen eine hohe Flexibilität bezüglich Größe und Form der zu präparierenden Knorpel geschaffen ist, und dass es auch möglich ist, bei der Präparation den Knorpel umgebende Knorpelhaut an diesem zu belassen.

Erfindungsgemäß wird die Aufgabe dadurch gelöst, dass der zweite Körper zumindest eine durch diesen hindurchgehende Öffnung aufweist, die als Aufnahme für den Knorpel dient, wobei den Knorpel umgebende Knorpelhaut zwischen den Körpern haltbar ist, und dass ein Schneidewerkzeug vorhanden ist, mittels dem ein über die Öffnung in dem zweiten Körper vorragender Abschnitt eines Knorpels abtrennbar ist.

Die Aufnahme ist somit nicht mehr als eine in dem ersten Körper ausgesparte Ausnahme ausgebildet, sondern als in dem zweiten Körper ausgesparte durchgehende Öffnung. Dadurch ist es möglich, zwischen erstem und zweitem Körper flächig die den knorpel umgebende Knorpelhaut zu legen und zwischen diesen beiden Körpern zu halten. Der zu präparierende Knorpel reicht durch die Öffnung in dem zweiten Körper hindurch und er wird dadurch zugeschnitten, indem das über die Öffnung des zweiten Körpers hinausragende Stück abgeschnitten wird. Die Geometrie des Schneidewerkzeuges kann der entsprechenden Oberfläche des zweiten Körpers optimal angepasst sein, so dass mit einem Schnitt problemlos der Knorpel entsprechend präpariert werden kann. Da die den Knorpel umgebende Haut zwischen den ersten und zweiten Körpern gehalten ist, kann diese nicht mehr mit dem Schneidewerkzeug in Berührung treten, so dass die Präparation ohne Beeinträchtigung der Knorpelhaut möglich ist.

Durch die Wahl der Form, der Größe und der Dicke des zweiten Körpers kann dann individuell auf die gewünschte Knorpelgröße angepaßt werden.

Dazu ist es vorteilhaft, daß der zweite Körper mehrere Öffnungen unterschiedlicher Größe und/oder unterschiedlicher Form aufweist.

Die Öffnungen können kreisrund, oval oder sonstwie geformt sein, wie es gerade gewünscht ist.

Dazu ist besonders vorteilhaft, daß das zweite Körper als plattenförmige Schablone ausgebildet ist.

Diese Maßnahme hat den Vorteil, daß die Schablone einfach auf den ersten Körper auflegbar ist und durch deren Öffnungen die zu präparierenden, sprich zuzuschneidenden Knorpel hervor ragen. Die Knorpelhaut ist flächig und sicher vor dem Schneidewerkzeug zwischen den beiden Körpern fixiert.

Die Variation der Dicke des Knorpels wird ganz einfach dadurch erreicht, daß mehrere zweite Körper unterschiedlicher Dicke vorhanden sind.

Es ist auch möglich, all diese Parameter bei einer einzigen Platte bzw. Schablone zu verwirklichen je nachdem, wie groß die Vorrichtung ausgeführt ist, also eine Schablone mit unterschiedlich geformten Löchern, mit unterschiedlich großen Löchern und mit unterschiedlich dicken Bereichen.

In einer weiteren Ausgestaltung der Erfindung ist der erste Körper als Block ausgebildet, auf dessen flächiger Oberseite der zweite Körper legbar ist.

Diese Maßnahme hat den Vorteil, daß für den präparierenden Arzt eine einfach handhabbare Vorrichtung vorhanden ist, die auch dem Schneidedruck beim Schneiden widersteht. Bei der eigentlichen Präparation wird das noch nicht zugeschnittene Knorpelstück samt der diesen umgebenden Knorpelhaut auf die flächige Oberseite des Blockes gelegt, anschließend darauf die flächige Schablone, und anschließend wird durch einen einfachen Schneidevorgang, bei dem die Schneidklingen über den oberen zweiten Körper geführt werden, der Knorpel entsprechend abgeschnitten, also das Stück, das durch die Öffnung des zweiten Körpers hinaus- und diesen überragt.

In einer weiteren Ausgestaltung der Erfindung sind erste und zweite Körper lagefest zueinander aufeinander legbar. Diese Maßnahme hat den Vorteil, daß bei dem eigentlichen Schneidevorgang, bei dem doch eine gewisse Kraft ausgeübt wird, die beiden Körper sich nicht relativ zueinander verschieben.

Die lagefeste Anordnung kann durch unterschiedliche Maßnahmen wie Klemmen, Rasten oder dgl. geschehen, eine sehr einfache Ausführungsform besteht darin, daß vom ersten Körper zumindest zwei Zapfen vorstehen, über die der zweite Körper, der dazu entsprechende durchgehende Bohrungen aufweist, aufsteckbar ist.

In einer weiteren Ausgestaltung der Erfindung sind im ersten Körper Saugöffnungen ausgespart, die zu der Seite münden, auf die der zweite Körper legbar ist.

Diese Maßnahme hat den Vorteil, daß über die Saugöffnungen ein auf die Oberseite des ersten Körpers aufgelegtes Transplantat ansaugbar ist. Dazu sind die Saugöffnungen mit einer entsprechenden Unterdruckquelle verbunden.

In einer weiteren Ausgestaltung der Erfindung ist ein Niederhalter vorhanden, mittels dem ein zwischen ersten und zweiten Körper und durch eine Öffnung im letzteren durchreichender Knorpel niederhaltbar ist.

Diese Maßnahme hat den Vorteil, daß beim eigentlichen Schneidevorgang der Knorpel, also das Stück, das über die Öffnung im zweiten Körper überragt, niedergehalten wird. Dadurch kann ausgeschlossen werden, daß durch das Schneidewerkzeug der Knorpel aus der Öffnung herausgezogen wird.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele im Zusammenhang mit den beiliegenden Zeichnungen näher beschrieben und erläutert.

Es zeigen:
- Fig. 1: perspektivisch eine Explosionsdarstellung eines ersten Ausführungsbeispiels einer erfindungsgemäßen Vorrichtung,
- Fig. 2: einen Schnitt längs der Linie II-II in Fig. 1 im zusammengebauten Zustand der Vorrichtung beim Präparieren eines Knorpels,
- Fig. 3: eine Draufsicht auf eine Oberseite eines ersten Körpers eines zweiten Ausführungsbeispiels einer erfindungsgemäßen Vorrichtung, mit einer Ansaugvorrichtung, und
- Fig. 4: einen Schnitt längs der Linie IV-IV in Fig. 3.

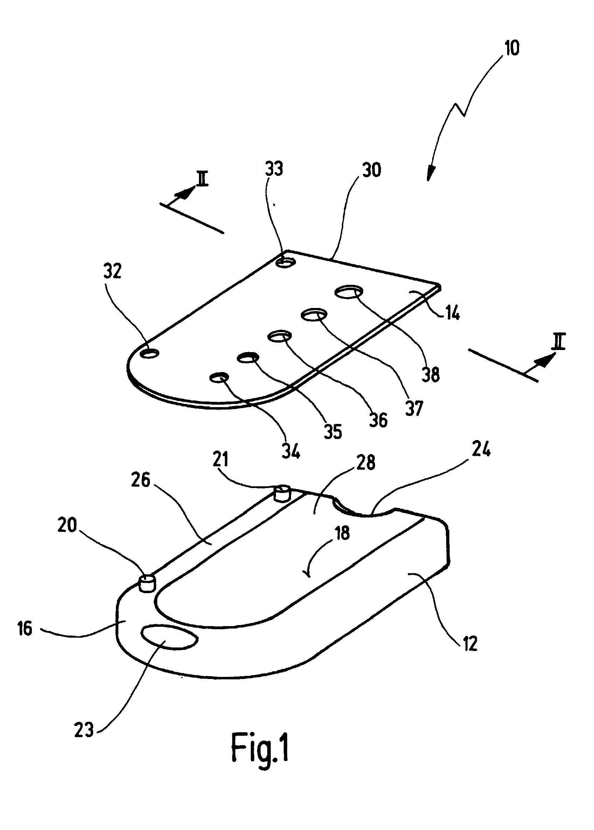

In den Fig. 1 bis 2 ist eine Vorrichtung zum Zuschneiden eines Knorpels 44 in ihrer Gesamtheit mit der Bezugsziffer 10 bezeichnet.

Die Vorrichtung 10 weist einen ersten Körper 12 und einen auf diesen auflegbaren zweiten Körper 14 auf.

Der erste Körper 12 besteht aus einem massiven Block 16, auf dessen ebener Oberseite 18 der zweite Körper 14 auflegbar ist.

Von der Oberseite 18 des Blockes 16 stehen an dessen Rand zwei Zapfen 20 und 21 vor, die als Lagefixierung für einen auf diese aufgeschobenen zweiten Körper 14 dient, wie es nachfolgend noch beschrieben wird.

An gegenüberliegenden Stirnseiten sind in der Oberseite Fingermulden 23 und 24 ausgespart, die dazu dienen, ein Abheben eines auf die Oberseite 18 aufgelegten zweiten Körpers 14 nach dem Handhabungsvorgang zu erleichtern.

Der Block 16 ist aus einem U-förmigen Rahmen 26 aufgebaut, in dem eine Schneideplatte 28 fixiert ist.

Der zweite Körper 14 ist als plattenförmige Schablone 30 ausgebildet, die seitlich zwei durchgehende Bohrungen 32 und 33 aufweist, über die die Schablone 30 auf die beiden Zapfen 20 und 21 aufgesteckt werden kann.

In der Schablone 30 sind an eine Reihe an durchgehenden, in dem dargestellten Ausführungsbeispiel kreisförmigen, Öffnungen 34 bis 38 vorgesehen, die unterschiedliche Durchmesser aufweisen.

Die Durchmesser variieren dabei von 6 bis 10 mm.

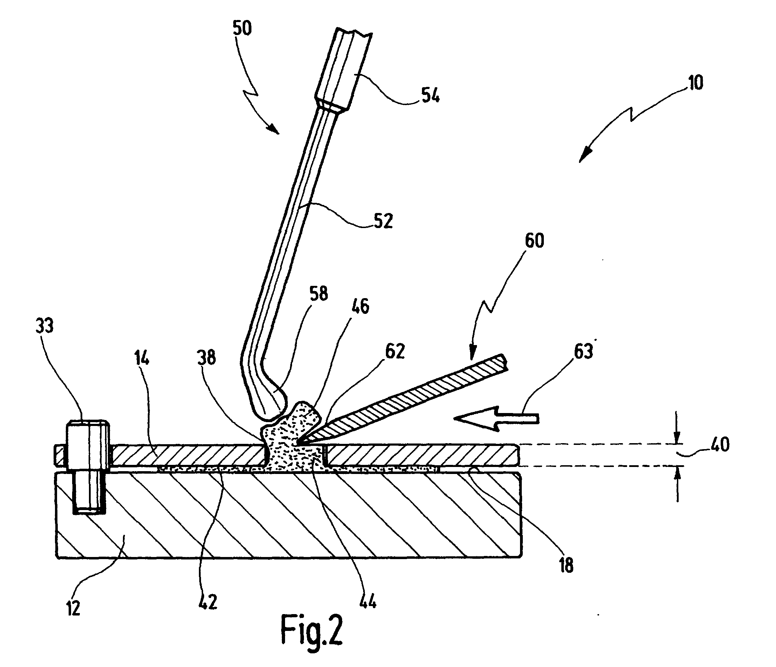

In Fig. 2 ist eine Situation dargestellt, bei der zunächst auf die Oberseite 18 des Blockes 16 ein Abschnitt einer Knorpelhaut 42 gelegt ist, von der einseitig ein Knorpel 44 hochsteht. Der Knorpel 44 wird nun so gelegt, daß er, wie es in Fig. 2 dargestellt ist, durch die Öffnung 38 hindurch ragt, wenn die plattenförmige Schablone 30 auf den Block 16 aufgelegt ist. Der Knorpel 44 hat eine solche Höhe, daß ein Abschnitt 46 die Oberseite der plattenförmigen Schablone 38 überragt.

Mit der Vorrichtung soll nun der Knorpel 44 auf eine Höhe zugeschnitten werden, die der Dicke 40 der Schablone 30 entspricht. Soll die Höhe des Knorpels 44 geringer oder größer sein, werden entsprechend dünnere oder dickere Schablonen 30 aufgelegt.

Aus der Schnittdarstellung von Fig. 2 ist auch zu erkennen, daß die den Knorpel 44 umgebende Knorpelhaut 42 flächig zwischen der Oberseite 18 des Blockes 16 und der hier nicht näher bezeichneten Unterseite der Schablone 30 zum Liegen kommt.

Beim eigentlichen Schneidevorgang wird ein Schneidewerkzeug 60 über die Oberseite der Schablone 30 geführt, wie es in Fig. 2 durch einen Pfeil 63 dargestellt ist, wobei dessen Schneide 62 den über die Schablone 30 vorragenden Abschnitt 46 des Knorpels 44 abtrennt.

Um sicherzustellen, daß der Knorpel 44 nicht durch das Schneidewerkzeug 60 aus der Öffnung 38 herausgezogen wird, ist noch ein Niederhalter 50 vorgesehen.

Der Niederhalter 50 weist einen lang erstreckten Schaft 52 der einerseits mit einem Griff 54 und andererseits mit einem abgewinkelten Stößel 56 versehen ist.

Der Stößel 56 ist so ausgebildet, daß er auf den Knorpel 44 aufgelegt werden kann und diesen niederhält.

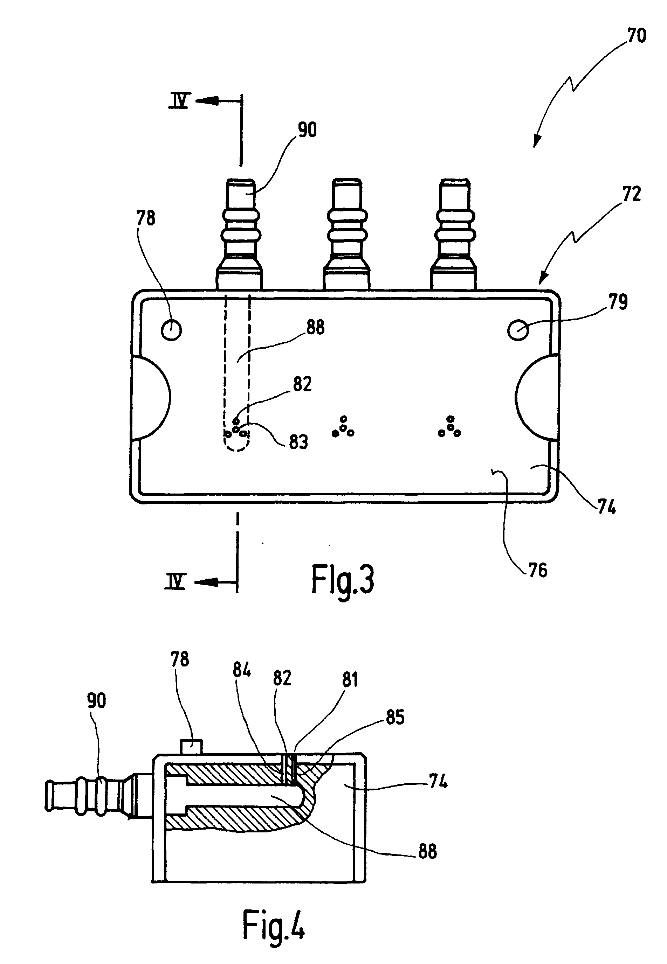

In den Fig. 3 und 4 ist ein weiteres Ausführungsbeispiel einer erfindungsgemäßen Vorrichtung dargestellt, die in ihrer Gesamtheit mit der Bezugsziffer 70 versehen ist.

In den Fig. 3 und 4 ist lediglich der erste Körper 72 dargestellt. Auch hier ist dementsprechend ein zweiter Körper vorhanden, der entsprechend wie der erste ausgebildet ist, also eine plattenförmige Schablone mit den entsprechend geformten Öffnungen.

Wie aus der Draufsicht von Fig. 3 ersichtlich, münden an der Oberseite 76 des ebenfalls als Block 74 ausgebildeten ersten Körpers 72 mehrere Saugöffnungen, wobei hier nur die zwei Saugöffnungen 82 und 83 bezeichnet sind. Von der Oberseite 76 springen auch wieder die beiden Zapfen 78 und 79 vor, um die darauf aufzulegende Schablone lagegerecht zu fixieren.

Aus der Schnittdarstellung von Fig. 4 ist zu erkennen, daß die Saugöffnungen 82 und 83 über Kanäle 84 und 85 mit einer Bohrung 88 in Verbindung stehen, die wiederum mit einem seitlichen Sauganschluß 90 in Verbindung steht. Über diesen Anschluß 90 kann der Block 74 an eine Unterdrucksaugvorrichtung angeschlossen werden. Wird also im Bereich der Saugöffnungen 82 und 83 ein Knorpel mit diesem umgebender Knorpelhaut aufgelegt, wird dieses Stück durch den angelegten Unterdruck gehalten.

Aus der Darstellung von Fig. 3 ist ersichtlich, daß mehrere solche Saugöffnungen bzw. entsprechende Bohrungsanschlüsse vorgesehen sind, die dann entsprechend so positioniert sind, daß sie unter den Öffnungen in der hier nicht dargestellten plattenförmigen Schablone liegen.

Es kann hier ausreichend sein, daß durch die Fixierung über den Unterdruck kein Niederhalter mehr notwendig ist. Es kann aber auch aus Sicherheitsgründen oder aus sonstigen Gründen durchaus auch noch ein Niederhalter 50 bei dem Schneidevorgang verwendet werden.

## Patentansprüche

1. Vorrichtung zum Zuschneiden von Knorpeln (44), mit einem ersten Körper (12, 72) und einem zweiten Körper (14), zwischen die der zuzuschneidende Knorpel (44) legbar ist, und mit einer Aufnahme, in der der Knorpel (44) aufnehmbar ist, **dadurch gekennzeichnet, daß** der zweite Körper (14) zumindest eine durch diesen hindurchgehende Öffnung (34-38) aufweist, die als Aufnahme für den Knorpel (44) dient, wobei den Knorpel (44) umgebende Knorpelhaut (42) zwischen den Körpern (12, 72; 14) haltbar ist, und daß ein Schneidewerkzeug (60) vorhanden ist, mittels dem ein über die Öffnung (38) in dem zweiten Körper (14) vorragender Abschnitt (46) eines Knorpels (44) abtrennbar ist.

2. Vorrichtung nach Anspruch 1, **dadurch gekennzeichnet, daß** der zweite Körper (14) mehrere Öffnungen (34-38) unterschiedlicher Größe und/oder unterschiedlicher Form aufweist.

3. Vorrichtung nach Anspruch 1 oder 2, **dadurch gekennzeichnet, daß** der zweite Körper (14) als plattenförmige Schablone (30) ausgebildet ist.

4. Vorrichtung nach einem der Ansprüche 1 bis 3, **dadurch gekennzeichnet, daß** mehrere zweite Körper (14) unterschiedlicher Dicke (40) vorhanden sind.

5. Vorrichtung nach einem der Ansprüche 1 bis 4, **dadurch gekennzeichnet, daß** der erste Körper (12, 72) als Block (16, 74) ausgebildet ist, auf dessen flächiger Oberseite (18, 76) der zweite Körper (14) legbar ist.

6. Vorrichtung nach Anspruch 5, **dadurch gekennzeichnet, daß** erster (12, 72) und zweiter Körper (14) lagefest zueinander aufeinander legbar sind.

7. Vorrichtung nach einem der Ansprüche 1 bis 6, **dadurch gekennzeichnet, daß** im ersten Körper (72) Saugöffnungen (82, 83) ausgespart sind, die zu der Seite münden, auf die der zweite Körper (14) legbar ist.

8. vorrichtung nach einem der Ansprüche 1 bis 7, **dadurch gekennzeichnet, daß** ein Niederhalter (50) vorhanden ist, mittels dem ein zwischen erstem (12) und zweitem Körper (14) und durch eine Öffnung (38) im letzteren durchragenden Knorpel (44) niederhaltbar ist.

## Claims

1. Device for cutting cartilage (44) to size, with a first body (12, 72) and a second body (14) between which the cartilage (44) to be cut to size can be placed, and with a seat in which the cartilage (44) can be received, **characterized in that** the second body (14) has at least one opening (34-38) extending through it, which opening (34-38) serves as a seat for the cartilage (44), and **in that** the perichondrium (42) surrounding the cartilage (44) can be held between the bodies (12, 72; 14), and **in that** a cutting tool (60) is provided by means of which a portion (46) of a cartilage (44) protruding through an opening (38) in the second body (14) can be cut off.

2. Device of claim 1, **characterized in that** the second body (14) has a plurality of openings (34-38) of different size and/or different shape.

3. Device of claims 1 or 2, **characterized in that** the second body (14) is designed as a plate-shaped stencil (30).

4. Device of anyone of claims 1 to 3, **characterized in that** a plurality of second bodies (14) of different thickness (40) are provided.

5. Device of anyone of claims 1 to 4, **characterized in that** the first body (12, 72) is designed as a block (16, 74) on whose flat upper face (18, 76) the second body (14) can be placed.

6. Device of claim 5, **characterized in that** the first body (12, 72) and second body (14) can be placed one on top of the other and in such a way as to be fixed in position relative to one another.

7. Device of anyone of claims 1 to 6, **characterized in that** suction openings (82, 83) are cut out in the first body (72) and open out towards the face on which the second body (14) can be placed.

8. Device of anyone of claims 1 to 7, **characterized in that** a holding-down tool (50) is provided by means of which a cartilage (44) located between the first body (12) and second body (14) and protruding through an opening (38) in the latter can be held down.

## Revendications

1. Dispositif pour couper des cartilages (44), comprenant un premier corps (12, 72) et un deuxième corps (14) entre lesquels peut être placé un cartilage (44) à couper, et comportant un logement dans lequel peut être reçu le cartilage (44), **caractérisé en ce que** le deuxième corps (14) présente au moins une ouverture (34-38) traversant celui-ci qui sert de logement au cartilage (44), la peau de cartilage (42) entourant le cartilage (44) pouvant être maintenue entre les corps (12, 72, 14), et **en ce qu'**il est prévu un outil de coupe (60) au moyen duquel on peut sectionner un tronçon (46) d'un cartilage (44) dépassant de l'ouverture (38) dans le deuxième corps (14).

2. Dispositif selon la revendication 1, **caractérisé en ce que** le deuxième corps (14) présente plusieurs ouvertures (34-38) de dimensions différentes et/ou de formes différentes.

3. Dispositif selon la revendication 1 ou 2, **caractérisé en ce que** le deuxième corps (14) est réalisé sous la forme d'un gabarit (30) en forme de plaque.

4. Dispositif selon l'une des revendications 1 à 3, **caractérisé en ce que** plusieurs corps (14) d'épaisseur (40) différente sont prévus.

5. Dispositif selon l'une des revendications 1 à 4, **caractérisé en ce que** le premier corps (12, 72) est réalisé sous la forme d'un bloc (16, 74) sur la face supérieure (18, 76) plate duquel peut être placé le deuxième corps (14).

6. Dispositif selon la revendication 5, **caractérisé en ce que** le premier corps (12, 72) et le deuxième corps (14) peuvent être placés l'un sur l'autre dans une position fixe l'un par rapport à l'autre.

7. Dispositif selon l'une des revendications 1 à 6, **caractérisé en ce que** dans le premier corps (72) sont découpées des ouvertures d'aspiration (82, 94) qui débouchent vers le côté sur lequel peut être placé le deuxième corps (14).

8. Dispositif selon l'une des revendications 1 à 7, **caractérisé en ce qu'**il est prévu un organe presseur (50) au moyen duquel on peut presser un cartilage (44) passant entre le premier corps (12) et le deuxième corps (14) et traversant une ouverture (38) de ce dernier.
